# EUROPEAN PATENT APPLICATION

(11) **EP 1 627 663 A2**
(43) Date of publication of application: **22.02.2006**
(21) Application number: 05010726.7
(22) Date of filing: 18.05.2005
(51) Int. Cl.: A61P 31/20, A61P 31/22, A61K 33/08, A61K 31/198, A61K 31/661

(54) **Use of a pharmaceutical composition for the treatment of pathologies derived from infection by herpes virus, papillomavirus, hepatitis virus, viral neuralgia, viral dsyplasia, immune-deficiency-induced viral infections and viral neuritis.**

(30) Priority: 18.05.2004 IT TO20040329
(71) Applicant: Deraco, Franco, 10137 TORINO (IT)
(72) Inventor: Deraco, Franco, 10137 TORINO (IT)
(74) Representative: Fiussello, Francesco

(57) **Abstract**

Use of a pharmaceutical composition including: 10-40% magnesium oxide, 10-40% DL-methionine, 0.1-5% calcium glycerophosphate, 0.1-3% ferric glycerophosphate, and 0.1-3% manganese glycerophosphate, for treating one of a group of pathologies including pathologies derived from infection by herpesvirus, pathologies derived from infection by papilloma virus, pathologies derived from infection by hepatitis virus, viral neuralgia, viral dysplasia, immune-deficiency-induced viral infections, and viral neuritis.

## Description

The present invention relates to use of a pharmaceutical composition comprising:
- 10-40% magnesium oxide
- 10-40% DL-methionine
- 0.1-5% calcium glycerophosphate
- 0.1-3% ferric glycerophosphate
- 0.1-3% manganese glycerophosphate
for the treatment of various pathologies.

Pathologies derived from herpesvirus infection are known to be treated using antiviral drugs, such as Aciclovir, which normally provide for reducing the course of the disease, pain, and complications, and for protecting immune-compromised subjects. Such drugs are sometimes administered in combination with corticosteroids or drugs containing capsaicin, to prevent or reduce the onset of inflammation, such as neuritis and consequent neuralgia; or antiinflammation drugs to relieve pain; or antihistaminic drugs to reduce itching.

Antiviral drugs, however, have numerous side-effects, such as nausea, vomiting, skin rashes, migraine, fatigue, trembling, and, more rarely, epileptic seizures.

Another powerful drug for the treatment of viral pathologies is Foscarnet, which is used in patients resistant to Aciclovir, but which also has serious side-effects, such as renal impairment and epileptic seizures.

Pathologies derived from infection by papilloma virus, and which are accompanied by the appearance at skin level of papules, nodules or excrescences, such as molluscum contagiosum, condylomas or vaginal epithelium dysplasia, are known to be treated by surgical removal or cryotherapy.

Besides being extremely painful, such treatment may result in scarring which, depending on the size and location of the area treated, may even result in physical impairment.

Pathologies derived from infection by hepatitis virus, such as hepatitis B and C, are known to be treated using drugs containing lamivudine, ribavirin or interferon. Interferon, in particular, may have side-effects, such as nausea, vomiting, fatigue, migraine, loss of appetite, and depression.

It is an object of the present invention to provide alternative treatments, with fewer side-effects, for the above viral infections.

According to the present invention, there is provided use of a pharmaceutical composition comprising:
- 10-40% magnesium oxide
- 10-40% DL-methionine
- 0.1-5% calcium glycerophosphate
- 0.1-3% ferric glycerophosphate
- 0.1-3% manganese glycerophosphate
for the treatment of pathologies selected from the group consisting of pathologies derived from infection by herpesvirus, pathologies derived from infection by papilloma virus, pathologies derived from infection by hepatitis virus, viral neuralgia, viral dysplasia, immune-deficiency-induced viral infections, and viral neuritis.

Said composition preferably comprises:
- 15-30% magnesium oxide
- 15-30% DL-methionine
- 1-3% calcium glycerophosphate
- 0.5-1.5% ferric glycerophosphate
- 0.5-1.5% manganese glycerophosphate

The composition even more preferably comprises:
- 22.5% magnesium oxide
- 25% DL-methionine
- 1.8% calcium glycerophosphate
- 0.9% ferric glycerophosphate
- 0.9% manganese glycerophosphate

In a preferred embodiment of the invention, in addition to the above components, the pharmaceutical composition also comprises at least one of vitamin B12 and vitamin E, which make the composition more effective and long-lasting.

"Pathologies derived from infection by herpesvirus" is intended to mean infections by viruses in the family Herpesviridae, e.g. by herpesvirus I, herpesvirus II, herpes zoster virus.

Among pathologies derived from infection by herpesvirus I, the composition according to the invention has proved particularly effective in the treatment of herpetic keratitis or herpes simplex.

Among pathologies derived from infection by herpesvirus II, the composition according to the invention has proved particularly effective in the treatment of genital herpes.

Among pathologies derived from infection by herpes zoster virus, the composition according to the invention has proved particularly effective in the treatment of chickenpox and herpes zoster.

"Pathologies derived from infection by papilloma virus" is intended to mean pathologies caused by infection by viruses in the papilloma virus family.

Among pathologies derived from infection by papilloma virus, the composition according to the invention has proved particularly effective in the treatment of condylomas, histiocytomas and papilloma-virus-induced psoriasis.

"Pathologies derived from infection by hepatitis virus" is intended to mean pathologies caused by infection by viruses in the hepatitis virus family.

Among pathologies derived from infection by hepatitis virus, the composition according to the invention has proved particularly effective in the treatment of hepatitis A, hepatitis B, and hepatitis C.

"Viral neuralgia" is intended to mean painful symptoms resulting from viral infection.

Among viral neuralgias, the composition according to the invention has proved particularly effective in the treatment of post-zoster neuralgia.

"Viral dysplasia" is intended to mean dysplasia resulting from viral infection.

Among viral dysplasias, the composition according to the invention has proved particularly effective in the treatment of vaginal epithelium dysplasia.

"Immune-deficiency-induced viral infections" is intended to mean pathologies caused by immune system impairment, e.g. caused by emotional stress, debilitating sickness or drugs, which facilitates the onset and development of viral infections.

The composition according to the invention has proved particularly effective in the treatment of HIV.

"Viral neuritis" is intended to mean pathologies caused by viral-infection-induced inflammation of the nerve sheath.

Among viral neurites, the composition according to the invention has proved particularly effective in the treatment of neuritis of the trigeminal nerve and facial nerve.

The composition according to the invention may be formulated in the usual way using one or more pharmaceutically acceptable excipients.

Non-limiting examples of pharmaceutical formulations are given below.

For use according to the invention, the composition may be formulated as a drug for topical, oral or transdermal administration.

More specifically, for topical administration, the composition may be prepared in the form of a flexible collodion solution containing excipients such as resin, castor oil, pyrosiline, ether and ethyl alcohol. Alternatively, the composition may be prepared in the form of a gel containing excipients such as lanolin Vaseline.

For oral administration, the pharmaceutical composition may be prepared, for example, in the form of tablets produced using known methods and pharmaceutically acceptable excipients, such as binding agents, fillers, additives, disintegrating and/or lubricating agents.

More preferably, for use according to the invention and for oral administration, the composition may be prepared, using known methods, in the form of coated tablets, e.g. of 1 gram.

For transdermal administration, the pharmaceutical composition may be prepared, for example, in the form of an transdermal patch comprising a gradual-release core containing salicylic acid (of 5 to 20% concentration).

A number of non-limiting features of the present invention are described below purely by way of example.

### EXAMPLE 1

### Treatment of condylomas

The following example refers to treatment of anal-genital condylomas using the composition according to the present invention.

For the testing, 100 patients suffering from anal-genital condylomas were selected and treated with a composition comprising:
225 mg magnesium oxide
250 mg DL-methionine
18 mg calcium glycerophosphate
9 mg ferric glycerophosphate
9 mg manganese glycerophosphate

The composition was administered orally in the form of coated 1-gram tablets, 2 tablets twice daily for 90 to 120 days, depending on the subject's ethnic type, epidermal turnover, and immune system condition. By the end of the above therapy, 90% of the patients were cured.

The procedure in Example 1 was also applied to the treatment of other pathologies, the results of which are shown in the examples below.

### EXAMPLE 2

### Treatment of genital herpes

Number of patients treated : 10
Duration of treatment : 60 days
Administration : oral
Formulation administered : the composition according to the invention in tablet form
Administrations per day : 2 tablets twice daily
Results : 90% cure

### EXAMPLE 3

Treatment of genital molluscum contagiosum
Number of patients treated : 50
Duration of treatment : 60-90 days
Administration : oral
Formulation administered : the composition according to the invention in tablet form
Administrations per day : 2 tablets twice daily
Results : 90% cure

### EXAMPLE 4

Treatment of herpetic keratitis
Number of patients treated : 2
Duration of treatment : 60-120 days
Administration : oral
Formulation administered : the composition according to the invention in tablet form
Administrations per day : 2 tablets twice daily
Results : 100% cure

### EXAMPLE 5

Treatment of post-zoster neuralgia
Number of patients treated : 3
Duration of treatment : 60-120 days
Administration : oral
Formulation administered : the composition according to the invention in tablet form
Administrations per day : 2 tablets twice daily
Results : 70% cure

### EXAMPLE 6

Treatment of labial herpes simplex I
Number of patients treated : 20
Duration of treatment : 60-120 days
Administration : oral
Formulation administered : the composition according to the invention in tablet form
Administrations per day : 2 tablets twice daily
Results : 100% cure

### EXAMPLE 7

Treatment of vaginal epithelium dysplasia
Number of patients treated : 50
Duration of treatment : 60-180 days (until negative pap test)
Administration : oral
Formulation administered : the composition according to the invention in tablet form
Administrations per day : 2 tablets twice daily
Results : 90% cure

### EXAMPLE 8

Treatment of papilloma-virus-induced psoriasis
Number of patients treated : 10
Duration of treatment : 60-120 days
Administration : oral
Formulation administered : the composition according to the invention in tablet form
Administrations per day : 2 tablets twice daily
Results : 90% cure

### EXAMPLE 9

Treatment of viral neuritis
Number of patients treated : 4
Duration of treatment : 60-180 days
Administration : oral
Formulation administered : the composition according to the invention in tablet form
Administrations per day : 2 tablets twice daily
Results : 75% cure

### EXAMPLE 10

Treatment of chickenpox
Number of patients treated : 2
Duration of treatment : 60 days
Administration : oral
Formulation administered : the composition according to the invention in tablet form
Administrations per day : 2 tablets twice daily
Results : 100% cure

## Claims

1. Use of a pharmaceutical composition comprising:
- 10-40% magnesium oxide
- 10-40% DL-methionine
- 0.1-5% calcium glycerophosphate
- 0.1-3% ferric glycerophosphate
- 0.1-3% manganese glycerophosphate
for the treatment of pathologies selected from the group consisting of pathologies derived from infection by herpesvirus, pathologies derived from infection by papilloma virus, pathologies derived from infection by hepatitis virus, viral neuralgia, viral dysplasia, immune-deficiency-induced viral infections, and viral neuritis.

2. Use as claimed in Claim 1, **characterized in that** said pharmaceutical composition comprises:
- 15-30% magnesium oxide
- 15-30% DL-methionine
- 1-3% calcium glycerophosphate
- 0.5-1.5% ferric glycerophosphate
- 0.5-1.5% manganese glycerophosphate

3. Use as claimed in Claim 1 or 2, **characterized in that** said pharmaceutical composition comprises:
- 22.5% magnesium oxide
- 25% DL-methionine
- 1.8% calcium glycerophosphate
- 0.9% ferric glycerophosphate
- 0.9% manganese glycerophosphate

4. Use as claimed in any one of the foregoing Claims, **characterized in that** said pharmaceutical composition comprises at least one of vitamin B12 and vitamin E.

5. Use as claimed in any one of Claims 1 to 4, for treatment of pathologies derived from infection by herpesvirus.

6. Use as claimed in Claim 5, **characterized in that** said pathology is derived from infection by herpesvirus I.

7. Use as claimed in Claim 6, **characterized in that** said pathology is herpetic keratitis or herpes simplex.

8. Use as claimed in Claim 5, **characterized in that** said pathology is derived from infection by herpesvirus II.

9. Use as claimed in Claim 8, **characterized in that** said pathology is genital herpes.

10. Use as claimed in Claim 5, **characterized in that** said pathology is derived from infection by herpes zoster virus.

11. Use as claimed in Claim 10, **characterized in that** said pathology is selected from the group consisting of chickenpox and herpes zoster.

12. Use as claimed in any one of Claims 1 to 4, for treatment of pathologies derived from infection by papilloma virus.

13. Use as claimed in Claim 12, **characterized in that** said pathology is selected from the group consisting of condylomas, histiocytomas and papilloma-virus-induced psoriasis.

14. Use as claimed in any one of Claims 1 to 4, for treatment of pathologies derived from infection by hepatitis virus.

15. Use as claimed in Claim 14, **characterized in that** said pathology is selected from the group consisting of hepatitis A, hepatitis B, and hepatitis C.

16. Use as claimed in any one of Claims 1 to 4, for treatment of viral neuralgia.

17. Use as claimed in Claim 16, **characterized in that** said neuralgia is post-zoster neuralgia.

18. Use as claimed in any one of Claims 1 to 4, for treatment of viral dysplasia.

19. Use as claimed in Claim 18, **characterized in that** said dysplasia is vaginal epithelium dysplasia.

20. Use as claimed in any one of Claims 1 to 4, for treatment of immune-deficiency-induced viral infections.

21. Use as claimed in any one of Claims 1 to 4, for treatment of viral neuritis.

22. Use as claimed in Claim 21, **characterized in that** said neuritis is selected from the group consisting of neuritis of the trigeminal nerve and neuritis of the facial nerve.

23. Use of the pharmaceutical composition as claimed in any one of the foregoing Claims administered in the form of tablets.

24. Use as claimed in Claim 23, **characterized in that** said tablets are coated tablets.

25. Use of the pharmaceutical composition as claimed in any one of Claims 1 to 22 administered in the form of an endermic plaster.

26. Use of the pharmaceutical composition as claimed in any one of Claims 1 to 22 administered in the form of gel.

27. Use of the pharmaceutical composition as claimed in any one of Claims 1 to 22 administered in the form of a flexible collodion solution.
